# EUROPEAN PATENT APPLICATION

(11) **EP 4 729 604 A1**
(43) Date of publication of application: **22.04.2026**
(21) Application number: 24822641.7
(22) Date of filing: 07.06.2024
(51) Int. Cl.: C12N 1/21, C12N 15/70, C12N 15/52, C12N 15/54, C12N 15/60, C12P 13/02, C12R 1/19

(54) **GENETICALLY ENGINEERED BACTERIUM FOR PRODUCING D-PANTOTHENIC ACID, CONSTRUCTION METHOD THEREFOR AND USE THEREOF**

(30) Priority: 13.06.2023 CN 202310695262
(71) Applicant: Zhejiang University of Technology, Hangzhou, Zhejiang 310014 (CN)
(72) Inventor: ZHANG, Bo, Hangzhou, Zhejiang 310014 (CN); HE, Zhoulin, Hangzhou, Zhejiang 310014 (CN); LIU, Zhiqiang, Hangzhou, Zhejiang 310014 (CN); TANG, Mengna, Hangzhou, Zhejiang 310014 (CN); XIAO, Yunying, Hangzhou, Zhejiang 310014 (CN); SHEN, Pan, Hangzhou, Zhejiang 310014 (CN); ZHENG, Yuguo, Hangzhou, Zhejiang 310014 (CN)
(74) Representative: Garavelli, Paolo
(86) International application number: PCT/CN2024/097912
(87) International publication number: WO 2024/255690

(57) **Abstract**

The present disclosure relates to genetically engineered bacteria for producing D-pantothenic acid, a construction method thereof, and application of the genetically engineered bacteria in preparation of the D-pantothenic acid through microbial fermentation. Mainly, (1) carbon flux is further directed toward the synthesis of the D-pantothenic acid by increasing the copy number of key genes in the synthesis pathway of pantothenic acid in an *Escherichia coli* (*E. Coli*) organism; and (2) in order to further enhance the carbon flux to direct toward the synthesis of the D-pantothenic acid, glycolysis front-end genes are activated by weakening a nitrogen limitation-negative regulatory transcription factor to drive the carbon flow, phosphoenolpyruvic acid is saved, the central carbon flow entering TCA is reduced, and a plasma-free and antibiotic-free genetically engineered bacterium strain for producing D-pantothenic acid is obtained. Finally, D-pantothenic acid shake flask titer is increased by 87.2% compared with an original strain, which reaches 5.43 g/L; and fermentation is performed with a 5 L fermentation tank for 84 hours, and the yield of the D-pantothenic acid can reach 94.2 g/L.

## Description

### TECHNICAL FIELD

The present disclosure relates to genetically engineered bacteria for producing D-pantothenic acid, a construction method, and application of the genetically engineered bacteria in preparation of the D-pantothenic acid through microbial fermentation.

### BACKGROUND ART

Pantothenic acid, also known as vitamin B5, is one of components of coenzyme A, which plays an important role in important biochemical reactions such as energy metabolism and citric acid circulation. Thus, as an important vitamin and precursor substance, D-pantothenic acid has been widely applied in feeds, medicines, cosmetics, etc. Among known D-pantothenic acid synthesis methods, a bio-fermentation method for producing D-pantothenic acid receives attention due to its advantages of cheap substrate, easy separation, low toxicity and the like. However, at present, there are still defects in producing D-pantothenic acid through a biological method, such as the problems of instable fermentation process, low yield and the like. Thus, it is still challengeable for constructing a D-pantothenic acid strain with a higher yield.

### SUMMARY

The present disclosure aims at providing a construction method of genetically engineered bacteria for producing D-pantothenic acid at a high yield through theoretical design and a CRISPR-Cas9 gene editing technology, and application of the genetically engineered bacteria in preparation of D-pantothenic acid through microbial fermentation.

The technical solutions of the present disclosure are as follows:
Genetically engineered bacteria for producing D-pantothenic acid, are constructed through the following method including:
(1) adopting genetically engineered bacteria ZJUTDPAL5 as chassis bacteria, increasing the copy number of an *EcilvD* gene regulated by a promoter pTrc on a genome of the genetically engineered bacteria, and obtaining an engineered bacteria DPAL6 derivative, *yjiV::*Ptrc*-EcilvD* and denoting the same as engineered bacteria DPAP7;
(2) increasing the copy number of a bacillus subtilis *BspanBA* gene regulated by the promoter pTrc on a genome of the engineered bacteria DPAP7 in a gene knockin manner, and obtaining an engineered bacteria DPAP7 derivative, *flik*::Ptrc-BspanBA and denoting the same as engineered bacteria DPAP8;
(3) increasing the copy number of a corynebacterium glutamicum *CgpanC* gene regulated by the promoter pTrc on a genome of the engineered bacteria DPAP8 in a gene knockin manner, and obtaining an engineered bacteria DPAP8 derivative, *ompT*::Ptrc-*CgpanC* and denoting the same as engineered bacteria DPAP9;
(4) increasing the copy number of a bacillus subtilis *alsS* gene regulated by the promoter pTrc on a genome of the engineered bacteria DPAP9 in a gene knockin manner, and obtaining an engineered bacteria DPAP9 derivative, *yjiP*::Ptrc*-alsS* and denoting the same as engineered bacteria DPAP10;
(5) further increasing the gene copy number of a bacillus subtilis *BspanBB* gene regulated by the promoter pTrc on a genome of the engineered bacteria DPAP10 in a gene knockin manner, and obtaining an engineered bacteria DPAP10 derivative, *ydeU::Ptrc-BspanBB* and denoting the same as engineered bacteria DPAP11; wherein *BspanBB* only differs from *BspanBA* in that a spacing between the promoter and a ribosome-binding site (rbs) is smaller, while sequences of *BspanB* are the same;
(6) replacing an initiator codon ATG of a *nac* gene in a genome of the engineered bacteria DPAP11 with GTG, and obtaining an engineered bacteria DPAP11 derivative, *nac*^{GTG} and denoting the same as engineered bacteria DPAP12;
(7) knocking out in-situ promoters P₃, P₄ and P₅ located upstream of gene clusters *ptsH, ptsI* and *crr* in a genome of the engineered bacteria DPAP12, and obtaining an engineered bacteria DPAP12 derivative, ΔP_{345ptsH} and denoting the same as engineered bacteria DPAP13;
(8) replacing an initiator codon ATG of a *gltA* gene in a genome of the engineered bacteria DPAP13 with GTG, and obtaining an engineered bacteria DPAP13 derivative, *gltA*^{GTG} and denoting the same as engineered bacteria DPAP14;
(9) replacing an initiator codon GTG of a *gltA* gene in a genome of the engineered bacteria DPAP14 with TTG, and obtaining an engineered bacteria DPAP14 derivative, *gltA*^{TTG} and denoting the same as engineered bacteria DPAP15; and
(10)replacing an in-situ promoter of a *pfkB* gene in a genome of the engineered bacteria DPAP15 with Ptrc, and obtaining an engineered bacteria DPAP15 derivative, PpfkB::Ptrc and denoting the same as engineered bacteria DPAP16, namely the genetically engineered bacteria for producing the D-pantothenic acid.

According to the present disclosure, on the basis of the chassis bacteria ZJUTDPAL5 (*E. coli* W3110, Trc-*panCpanEpanBilvC*/*ilvG** /Δ*avtA*/*ilvE**/*coaA**/Δ*ilvA*/Trc-*lpd*/Δ*glk*/*ilvA**/Trc-*pck*/Trc-*maeB*/Trc-*ilvBN*/*gdhA**^{T}, which has been disclosed in CN113637618A), by comprehensively applying a systematic metabolic engineering strategy and using the CRISPR/Cas9 gene editing technology, heterologous genes are introduced through gene knockin, an expression level of key genes in the synthesis pathway of pantothenic acid in an *E. coli* organism is enhanced, and carbon flux is further directed toward the synthesis of the D-pantothenic acid. In order to further enhance the carbon flux to direct toward the synthesis of the D-pantothenic acid, glycolysis front-end genes are activated by weakening a nitrogen limitation-negative regulatory transcription factor to drive the carbon flow, phosphoenolpyruvic acid is saved, the central carbon flow entering TCA is reduced, and a plasma-free and antibiotic-free genetically engineered bacterium strain for producing the D-pantothenic acid is obtained.

Nucleotide sequences of the *EcilvD, BspanBA, CgpanC, BspanBB* and *alsS* genes regulated by the promoter Ptrc are respectively shown in SEQ ID NO. 1-5, a nucleotide sequence of the *nac^{GTG}* is shown in SEQ ID NO. 6, nucleotide sequences of the in-situ promoters P₃, P₄ and P₅ located upstream of the gene clusters *ptsH, ptsI* and *crr* are shown in SEQ ID NO. 7, a nucleotide sequence of the *gltA*^{GTG} is shown in SEQ ID NO. 8, a nucleotide sequence of the *gltA*^{TTG} is shown in SEQ ID NO. 9, and a nucleotide sequence of the in-situ promoter of the *pfkB* gene is shown in SEQ ID NO. 10.

The present disclosure further relates to a construction method of the genetically engineered bacteria, including:
(1) adopting genetically engineered bacteria ZJUTDPAL5 as chassis bacteria, applying a CRISPR-Cas9 mediated gene editing technology, replacing an original pseudogene *yjiV* on a genome of an original strain with an *EcilvD* gene regulated by a promoter Ptrc derived from pTrc99A, so as to enhance expression intensity of *EcilvD,* and obtaining an engineered bacteria DPAL6 derivative, *yjiV::Ptrc-EcilvD* and denoting the same as engineered bacteria DPAP7;
(2) adopting the engineered bacteria DPAP7 as an original strain, applying the CRISPR-Cas9 mediated gene editing technology, replacing an original pseudogene *flik* on a genome of the original strain with a *BspanBA* gene regulated by the promoter Ptrc derived from pTrc99A, so as to enhance expression intensity of *BspanBA,* and obtaining an engineered bacteria DPAP7 derivative, *flik::Ptrc-BspanBA* and denoting the same as engineered bacteria DPAP8;
(3) adopting the engineered bacteria DPAP8 as an original strain, applying the CRISPR-Cas9 mediated gene editing technology, replacing an original pseudogene *ompT* on a genome of the original strain with a *CgpanC* gene regulated by the promoter Ptrc derived from pTrc99A, so as to enhance expression intensity of *CgpanC,* and obtaining an engineered bacteria DPAP8 derivative, *ompT::Ptrc-CgpanC* and denoting the same as engineered bacteria DPAP9;
(4) adopting the engineered bacteria DPAP9 as an original strain, applying the CRISPR-Cas9 mediated gene editing technology, replacing an original pseudogene *yjiP* on a genome of the original strain with an *alsS* gene regulated by the promoter Ptrc derived from pTrc99A, so as to enhance expression intensity of *alsS,* and obtaining an engineered bacteria DPAP9 derivative, *yjiP::Ptrc-alsS* and denoting the same as engineered bacteria DPAP10;
(5) adopting the engineered bacteria DPAP10 as an original strain, applying the CRISPR-Cas9 mediated gene editing technology, replacing an original pseudogene *ydeU* on a genome of the original strain with a *BspanBB* gene regulated by the promoter Ptrc derived from pTrc99A, so as to enhance expression intensity of *BspanBB,* and obtaining an engineered bacteria DPAP10 derivative, *ydeU::Ptrc-BspanBB* and denoting the same as engineered bacteria DPAP11;
(6) adopting the engineered bacteria DPAP11 as an original strain, applying the CRISPR-Cas9 mediated gene editing technology, replacing an initiator codon ATG of a *nac* gene in a genome of the engineered bacteria DPAP11 with GTG, and obtaining an engineered bacteria DPAP11 derivative, *nac*^{GTG} and denoting the same as engineered bacteria DPAP12;
(7) adopting the engineered bacteria DPAP12 as an original strain, applying the CRISPR-Cas9 mediated gene editing technology, knocking out in-situ promoters P3, P4 and P5 located upstream of gene clusters *ptsH, ptsI* and *crr* in a genome of the engineered bacteria DPAP12, and obtaining an engineered bacteria DPAP12 derivative, ΔP_{345ptsH} and denoting the same as engineered bacteria DPAP13;
(8) adopting the engineered bacteria DPAP13 as an original strain, applying the CRISPR-Cas9 mediated gene editing technology, replacing an initiator codon ATG of a *gltA* gene in a genome of the engineered bacteria DPAP13 with GTG, and obtaining an engineered bacteria DPAP13 derivative, *gltA*^{GTG} and denoting the same as engineered bacteria DPAP14;
(9) adopting the engineered bacteria DPAP14 as an original strain, applying the CRISPR-Cas9 mediated gene editing technology, replacing an initiator codon GTG of a *gltA* gene in a genome of the engineered bacteria DPAP14 with TTG, and obtaining an engineered bacteria DPAP14 derivative, *gltA*^{TTG} and denoting the same as engineered bacteria DPAP15; and
(10)adopting the engineered bacteria DPAP15 as an original strain, applying the CRISPR-Cas9 mediated gene editing technology, replacing an in-situ promoter of a *pfkB* gene in a genome of the engineered bacteria DPAP15 with Ptrc, and obtaining an engineered bacteria DPAP15 derivative, PpfkB::Ptrc and denoting the same as engineered bacteria DPAP16, namely the genetically engineered bacteria for producing the D-pantothenic acid.

Specifically, nucleotide sequences of the *EcilvD, BspanBA, CgpanC, BspanBB* and *alsS* genes regulated by the promoter Ptrc are respectively shown in SEQ ID NO. 1-5, a nucleotide sequence of the *nac*^{GTG} is shown in SEQ ID NO. 6, nucleotide sequences of the in-situ promoters P₃, P₄ and P₅ located upstream of the gene clusters *ptsH, ptsI* and *crr* are shown in SEQ ID NO. 7, a nucleotide sequence of *gltA*^{GTG} is shown in SEQ ID NO. 8, a nucleotide sequence of *gltA*^{TTG} is shown in SEQ ID NO. 9, and a nucleotide sequence of the in-situ promoter of the *pfkB* gene is shown in SEQ ID NO. 10.

The present disclosure further relates to application of the genetically engineered bacteria in preparation of D-pantothenic acid through microbial fermentation.

The application is as follows: Inoculating the genetically engineered bacteria for producing the D-pantothenic acid into a fermentation medium, performing fermentation culture at 28-37°C and 300-450 rpm for 72-96 hours, taking a supernatant of a fermentation broth after fermentation for separation and purification, and obtaining the D-pantothenic acid.

Specifically, the fermentation medium includes the following components: 10-30 g/L of glucose, 10-25 g/L of ammonium sulfate, 1-5 g/L of betaine anhydrous, 1-5 g/L of yeast powder, 1-5 g/L of potassium dihydrogen phosphate, 0.5-2 g/L of anhydrous magnesium sulfate, 1-5 g/L of beta-alanine and 1-5 mL/L of microelement solution, deionized water is adopted as a solvent, and a pH value is natural; and the microelement solution includes the following components: 10 g/L of CuCl₂, 10 g/L of FeSO₄·7H₂O, 10 g/L of ZnSO₄·7H₂O, 0.2 g/L of CuSO₄ and 0.02 g/L of NiCl₂·7H₂O, and deionized water is adopted as a solvent.

Specifically, a method includes: Filling a 5 L fermentation tank with the fermentation medium with a volume of 1-3 L, sterilizing at 115°C for 30 minutes, inoculating a strain of the genetically engineered bacteria into the fermentation medium with the volume of 1-3 L, performing fermentation culture at 28-37°C, initial ventilation capacity of 3-6 L/min, and initial stirring speed of 300-450 rpm, regulating pH with aqueous ammonia, and meanwhile, adding IPTG with a final concentration of 0-0.4 mM, VB₁ with a final concentration of 5 mg/L, VB₁₂ at 2 mg/L and 5-10 mL of isoleucine at 10-40 g/L; and maintaining dissolved oxygen at 10-30% at a dissolved oxygen series rotating speed during fermentation, maintaining pH at 6.7-6.9 with ammonium hydroxide as a neutralizer, adding a feeding medium into the tank through pH linked feeding, controlling a glucose concentration below 5 g/L, culturing at 28-37°C for 72-96 hours, obtaining the fermentation broth, taking the supernatant of the fermentation broth for separation and purification, and obtaining the D-pantothenic acid.

The feeding medium includes the following components: 500 g/L of glucose, 5-25 g/L of ammonium sulfate, 2-8 g/L of betaine anhydrous, 1-5 g/L of yeast powder, 10-20 g/L of potassium dihydrogen phosphate, 5-15 g/L of anhydrous magnesium sulfate, 40-100 g/L of beta-alanine and 1-5 mL/L of microelement solution, deionized water is adopted as a solvent, and a pH value is natural.

The copy number of *panC* (encoded pantothenate synthetase), *panB* (encoded hydroxymethyl transferases), *alsS* (encoded acetolactate synthase) and *ilvD* (encoded dihydroxy acid dehydratase) is further increased on a genome by the promoter pTrc derived from pTrc99A and an RBS sequence while heterologously screening pantothenic acid branch genes, the pantothenic acid synthesis pathway is enhanced, and DPAP11 (DPAP10 derivative, *ydeU*::Ptrc*-BspanBB*) is constructed.

The nitrogen limitation-negative regulatory transcription factor *nac* is weakened through the CRISPR/Cas9 gene editing technology, initiators of the genes *ptsH, ptsl, Crr* of a glucose phosphotransferase system are weakened, phosphoenolpyruvic acid is saved, citrate synthase *gltA* is knocked out, the central carbon flow entering TCA is reduced, the carbon flow is driven by activating glycolysis pathway front-end genes *pfkB,* and genetically engineered bacteria DPAP16 without plasmid and antibiotics added during fermentation are obtained.

Compared with the prior art, the present disclosure has the following main beneficial effects:
The expression level of key enzymes in the D-pantothenic acid bio-generation pathway is further enhanced on the basis of existing engineered bacteria by applying the CRISPR/Cas9 gene editing technology, the glycolysis pathway, TCA cycle and expression of one or more key genes in global cellular regulatory factors are up-regulated or down-regulated, and the plasmid-free high-yield strain without antibiotics added in the fermentation process is obtained. The shake flask titer of the strain reaches 5.43 g/L, which is increased by 87.2% compared with the original strain, fermentation is performed with the 5 L fermentation tank for 84 hours, the yield of the D-pantothenic acid can reach 94.2 g/L, the synthesis of the D-pantothenic acid is greatly improved, and the fermentation period is shortened.

### BRIEF DESCRIPTION OF THE DRAWINGS

FIG. 1 is a D-pantothenic acid metabolic pathway diagram and modification sites;
FIG. 2 shows an OD₆₀₀ and D-pantothenic acid titer change of DPAP7;
FIG. 3 shows an OD₆₀₀ and D-pantothenic acid titer change of DPAP8;
FIG. 4 shows an OD₆₀₀ and D-pantothenic acid titer change of DPAP9;
FIG. 5 shows an OD₆₀₀ and D-pantothenic acid titer change of DPAP10;
FIG. 6 shows an OD₆₀₀ and D-pantothenic acid titer change of DPAP11;
FIG. 7 shows an OD₆₀₀ and D-pantothenic acid titer change of DPAP12;
FIG. 8 shows an OD₆₀₀ and D-pantothenic acid titer change of DPAP13;
FIG. 9 shows an OD₆₀₀ and D-pantothenic acid titer change of DPAP14 and DPAP15;
FIG. 10 shows an OD₆₀₀ and D-pantothenic acid titer change of DPAP16; and
FIG. 11 is a fermentation result diagram of DPAP11 and DPAP16 in a 5 L bio-reactor.

### DETAILED DESCRIPTION OF THE EMBODIMENTS

The present disclosure will be further described with reference to specific examples, however, the scope of protection of the present disclosure is not limited thereto.

In the following examples, a final concentration of spectinomycin in a medium is 0.05 mg/L, and a final concentration of kanamycin in a medium is 0.05 mg/L.

A parent strain *E. coli* W3110 was obtained from Yale University CGSC Collection Center (Coli Genetic Stock Center), the collection date was August 5, 1975, and the collection number is CGSC#4474, and it had been disclosed in Patent US 2009/0298135 A1, US 2010/0248311 A1.

Information of primer sequences used in Examples 2-11 is shown in Table 2:

**Table 1: Genes involved in gene editing and corresponding pathways**

| Gene | Involved pathway |
|---|---|
| *EcilvD* | Pantothenic acid synthesis |
| *BspanB* | Pantothenic acid synthesis |
| *CgpanC* | Pantothenic acid synthesis |
| *alsS* | Pantothenic acid synthesis |
| *ptsHICrr* | Glucose phosphate transfer system |
| *pfkB* | Glycolysis pathway |
| *nac* | Global cellular regulatory factor |
| *gltA* | TCA cycle |

**Table 2: Primer sequence**

| Primer name | Sequence (5'-3') |
|---|---|
| *ilvD-VF* | TCAGCATCGGAAGGTTTGC |
| *ilvD-VR* | GACATCACCATTCGCTCATC |
| *pT-yjiV-F* | TAATACTAGTGATGAAGGCAAAGAGGGCGTGTTTTAGAGCTAGAAATAGC |
| pT*-yjiV-*R | GCTCTAAAACACGCCCTCTTTGCCTTCATCACTAGTATTATACCTAGGAC |
| *yjiV-*S6F | CGGTGCTTTTTTTGAATTCTCTAGAGGACTCAACCTAATGCAAAGG |
| *yjiV-*S6R | GTGAAACATTATACGAGCCGGATGATTAATTGTCAAACTGGTAAAGTCCTG ACAATACT |
| pTrc-*ilvD*-18F | AATCATCCGGCTCGTATAATGTTTCACACAGGAAACAGACCATGCCTAAGT ACCGTTCC |
| pTrc-*ilvD*-18R | CAGCGTCACTTCAGCGCCATCTTAACCCCCCAGTTTCGAT |
| *yjiV-*X6F | GATGGCGCTGAAGTGACG |
| *yjiV-*X6R | GGGTAATAGATCTAAGCTTCTGCACCACGTAGATACCGCAGTG |
| *BspanBA-*VF | *CTCACTGAAGGCGATGACC* |
| *BspanBA-*VR | *CATGGTACGGCTGAATGC* |
| pT*-flik-*F | TAATACTAGTCGAGACAACTACCGACAAAGGTTTTAGAGCTAGAAATAGC |
| pT*-flik-*R | GCTCTAAAACCTTTGTCGGTAGTTGTCTCGACTAGTATTATACCTAGGAC |
| *flik*-S8F | CGGTGCTTTTTTTGAATTCTCTAGAACGGCGTTGATCAGTGAG |
| *flik-*S8R | TTATCCGCTCACAATTCCACACATTATACGAGCCGGATGATTAATTGTCAAC CTGCTAATGCTTCGCTC |
| pTrc*-BspanBA-*9F | TGTGTGGAATTGTGAGCGGATAACAATTTCACACAGGAAACAGACCATGA AAACAAAACTGGATTTTCT |
| pTrc-*BspanBA-*9R | CCAGAGGTTCATGGTTTGCTGTTTATTTTCCCCCGTACAAGC |
| *flik-*X8F | GCTTGTACGGGGGAAAATAAACAGCAAACCATGAACCTCT |
| *flik-*X8R | GGGTAATAGATCTAAGCTTCTGCAGGGTATTCGGATCGTACGGAC |
| *CgpanC-*VF | *GTTTAAGCCCCAGCCTGC* |
| *CgpanC-*VR | *GAATGGCTATATTGCTGAAGAGG* |
| pT-*ompT*-F | TAATACTAGTTACTCCTGACAACATAAATGGTTTTAGAGCTAGAAATAGC |
| pT-*ompT*-R | GCTCTAAAACCATTTATGTTGTCAGGAGTAACTAGTATTATACCTAGGAC |
| *ompT*-S6F | CGGTGCTTTTTTTGAATTCTCTAGACCTGACACCGTTGAATTATCTCG |
| *ompT*-S6R | TGTGAAACATTATACGAGCCGGATGATTAATTGTCAAAACGATAAAGTCTC GGTAGAAGC |
| pTrc*-CgpanC-*8F | AATTAATCATCCGGCTCGTATAATGTTTCACACAGGAAACAGACCATGCAG GTAGCAACCACAAAG |
| pTrc*-CgpanC-*8F | CCATTTTTGCTGTAGTCTGATTAGAGCTCGATATTGTCGATCAAC |
| *ompT*-X6F | TAATCAGACTACAGCAAAAATGGAGC |
| *ompT*-X6R | GGGTAATAGATCTAAGCTTCTGCAGGCAATGGCATTTAAAAGATATTGCG |
| *alsS-*VF | ACGTTGTGAACATTGTCGCGG |
| *alsS-*VR | ATAGCGGCGAAGCGCCTG |
| *yjip-*S5F | CGGTGCTTTTTTTGAATTCTCTAGACATACAAAATCAACGCCATCGGG |
| *yjip-*S5R | GTGTGAAACATTATACGAGCCGGATGATTAATTGTCAAGGCGATCCATCAC CTGTTGA |
| *yjip*-pT-F | TAATACTAGTGCTTTGTCGATGAAAAATTGGTTTTAGAGCTAGAAATAGC |
| *yjip-*pT-R | GCTCTAAAACCAATTTTTCATCGACAAAGCACTAGTATTATACCTAGGAC |
| pTrc*-alsS-*17F | TCCGGCTCGTATAATGTTTCACACAGGAAACAGACCTTGACAAAAGCAAC AAAAGAACAAAAATCCC |
| pTrc*-alsS*-17R | GTTTATCATGCTCTATATGGCGCTAGAGAGCTTTCGTTTTCATGAGTTCC |
| *yjip-*X6F | AAGCTCTCTAGCGCCATATAGAGCATGATAAACGCC |
| *yjip-*X6R | GGGTAATAGATCTAAGCTTCTGCAGCGTCTCTGATTAGCTCTTGAGCC |
| *BspanBB-*VF | GGCGAACTGCGTAATAGC |
| *BspanBB-*VR | CTGGTTGTTCATCCAAGTGAC |
| pT*-ydeU*-F | TAATACTAGTTGGGGCTTACGTCTACACGCGTTTTAGAGCTAGAAATAGC |
| pT*-ydeU-*R | GCTCTAAAACGCGTGTAGACGTAAGCCCCAACTAGTATTATACCTAGGAC |
| *ydeU-*S6F | CGGTGCTTTTTTTGAATTCTCTAGACCTGCTCACCATCTCCACT |
| *ydeU-*S6R | GAAATTCCACACATTATACGAGCCGGATGATTAATTGTCAAGCTTCGACAG TTCCGGTG |
| pTrc-*BspanB*B-9F | CGTATAATGTGTGGAATTTCACACAGGAAACAGACCATGAAAACAAAACT GGATTTTCT |
| pTrc-BspanBB-9R | CGCTGGTGATGGCTGTTCAGTTATTTTCCCCCGTACAAGCC |
| *ydeU-X6F* | CTGAACAGCCATCACCAG |
| *ydeU-X6R* | GGGTAATAGATCTAAGCTTCTGCAGCGTCGCCTGCTTAATACC |
| pTarget-XF | GCAGGTCGACTCTAGAGAATTC |
| pTarget-XR | GAAGCTTAGATCTATTACCCTG |
| pT-*nac-F* | TAATACTAGTAGATATTGGTAGCCTGACCCGTTTTAGAGCTAGAAATAGC |
| pT*-nac-R* | GCTCTAAAACGGGTCAGGCTACCAATATCTACTAGTATTATACCTAGGAC |
| nac-up-F | ATTCTCTAGAGTCGACCTGCAACGGGCAAGAAGTTGATGTAAAT |
| nac-up-R | TCGACAATTTTTACAAAGTATTTCAGGCGTCTGAAGTTCACGTTGCC |
| nac-down-F | TACTTTGTAAAAATTGTCGACATCGGATCCCTGACGCAGGCTGCTGAAGTA T |
| nac-down-R | GGGTAATAGATCTAAGCTTCTTAGCTCACCAATTGCCACTG |
| pT-PptsH-F | TAATACTAGTTGCGCGAAATTAATCGTTACGTTTTAGAGCTAGAAATAGC |
| pT-PptsH-R | GCTCTAAAACGTAACGATTAATTTCGCGCAACTAGTATTATACCTAGGAC |
| PptsH-up-F | ATTCTCTAGAGTCGACCTGCGGGACTGGCGGTACGCTGAC |
| PptsH-up-R | CAGCTTTGGCGGCCACAAAAAAGCACCT |
| PptsH-down-F | TTTTGTGGCCGCCAAAGCTGAATCGATTTTATG |
| PptsH-down-R | GGGTAATAGATCTAAGCTTCCTACCTTACTTGTGACTGATTT |
| pT-gltA-F | TAATACTAGTGCTGATCGATCGGGAAACCGGTTTTAGAGCTAGAAATAGC |
| pT-gltA-R | GCTCTAAAACCGGTTTCCCGATCGATCAGCACTAGTATTATACCTAGGAC |
| gltA-up-F | ATTCTCTAGAGTCGACCTGCTTCCAGCATTTTCAGCGCC |
| gltA-up-R | TGGCGACCGATTCTAACTAC |
| gltA-gRNA-F | GTAGTTAGAATCGGTCGCCAGCTGATCAATCGGAAAGCCACGGTGCAGCA AAATACCTT |
| gltA-gRNA-R | GGCTGATACAAAAGCAAAACTC |
| gltA-down-F1 | AGTTTTGCTTTTGTATCAGCCACTTAAGGTCTCCTTAGCGCCT |
| gltA-down-F2 | AGTTTTGCTTTTGTATCAGCCAATTAAGGTCTCCTTAGCGCCT |
| gltA-down-R | GGGTAATAGATCTAAGCTTCGCGTCCTTTCTATAACTGCG |
| pT-PpfkB-F | TAATACTAGTTCAGTCTGGCACCGAATCAGGTTTTAGAGCTAGAAATAGC |
| pT-PpfkB-R | GCTCTAAAACCTGATTCGGTGCCAGACTGAACTAGTATTATACCTAGGAC |
| PpfkB-up-F | ATTCTCTAGAGTCGACCTGCATTTTTCTGAAGAACGCTCATC |
| PpfkB-up-R | TCCACACATTATACGAGCCGGATGATTAATTGTCAAGCTTTATTTAAAATTT TGCAGAT |
| PpfkB-down-F | GCTCGTATAATGTGTGGAATTTTTCACACAGGAAACAGACCATGGTACGTA TCTATACG |
| PpfkB-down-R | GGGTAATAGATCTAAGCTTCCTGTCGACGATGCAGCGG |

### Example 1: HPLC assay for D-pantothenic acid content

A detection method is as follows:
Chromatographic conditions: C₁₈ column (250 × 4.6 mm, particle size 5 µm, Agilent Technologies Co., Santa Clara, CA, USA), detection wavelength: 200 nm, and column temperature: 30°C;
Sample treatment: A sample was diluted with ultra-pure water, and D-pantothenic acid content was kept at 0.05 g/L-0.40 g/L;
mobile phase: Acetonitrile/water/phosphoric acid: (50/949/1);
data acquisition time: 25 minutes.

### Example 2: DPAP7 (DPAL6 derivative, yjiV::Ptrc-EcilvD) construction and shake flask fermentation

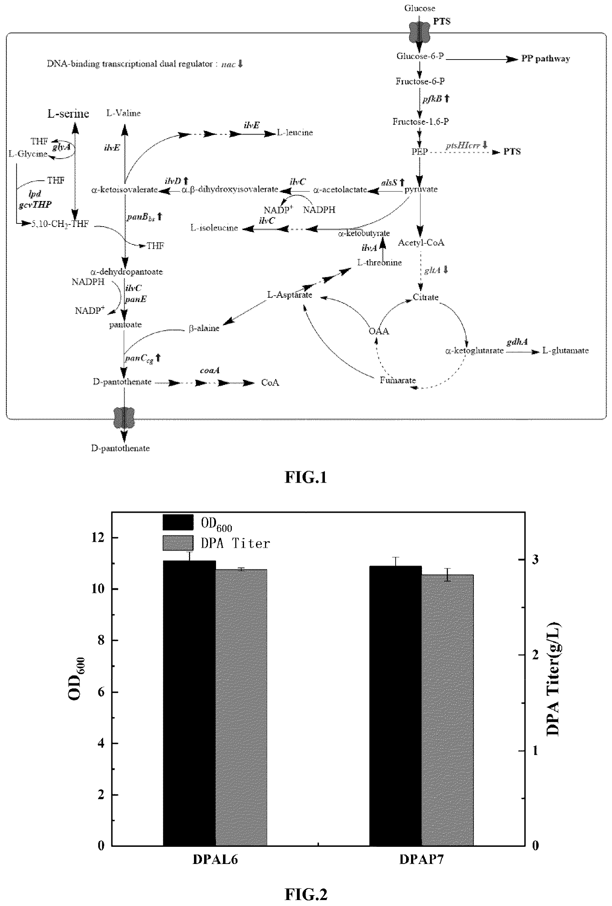

ZJUTDPAL5 (*E. coli* W3110, *Trc-panCpanEpanBilvC*/*ilvG**/*ΔavtA*/*ilvE**/*coaA**/*ΔilvA*/Trc*-lpd*/*Δglk*/*ilvA**/Trc-*pck*/ *Trc-maeB*/ Trc-*ilvBN*/*gdhA**^{T}) was adopted as an original strain, a CRISPR-Cas9 mediated gene editing technology was applied, and an original pseudogene ydeU on a genome of the original strain was replaced with an *EcilvD* gene (nucleotide sequence was shown in SEQ ID No. 1) regulated by a promoter Ptrc derived from pTrc99A, so as to enhance expression intensity of *EcilvD.*
(1) Construction of plasmid pTarget-*yjiV*: A pTarget F plasmid (Addgene Plasmid #62226) was adopted as a template, pT*-yjiV*-F/R was adopted as primers for PCR amplification, a nucleic acid PCR product was digested with a *Dpn* I digestive enzyme at a temperature preserved at 37°C for 3 hours after gel electrophoresis verification and then transferred to *E. coli* DH5α, and a correct pTarget-*yjiV* plasmid was obtained after spectinomycin plate screening and sequencing verification, which was used for ligation with donor DNA subsequently.
(2) Construction of plasmid *pTD-EcilvD:* Firstly, an *E. coli* W3110 genome was adopted as a template, *yjiV*-S6F/R was adopted as primers for amplification to obtain an upstream portion (F1) of the donor DNA, and *yjiV*-X6F/R was adopted as primers for amplification to obtain a downstream portion (F2) of the donor DNA. Then, an *E .coli* W3110 genome was adopted as a template, pTrc-EcilvD-18F/R was adopted as primers for amplification to obtain an *EcilvD* gene fragment (F3) with a promoter pTrc, and PCR fragments were recycled and purified with glue to obtain F1, F2 and F3; the plasmid pTarget-*yjiV* was subjected to heat preservation at 37°C for 8 hours through *Xba* I and *Pst* I*,* and DNA fragments were recycled with a Clean up kit; the fragments F1, F2 and F3 were ligated into the carrier pTarget-*yjiV*according to the package insert of ClonExpress^{®} (One step clone kit, Vazyme Biotech, Nanjing, China), and a plasmid pTD*-EcilvD* was obtained after sequencing verification.
(3) A pCas plasmid (Addgene Plasmid #62225) was introduced into ZJUTDPAL5 *(E. coli* W3110,
   Trc-*panCpanEpanBilvC*/*ilvG**/*ΔavtA*/*ilvE**/*coaA**/*ΔilvA*/Trc*-lpd*/*Δglk*/*ilvA**/Trc-*pck*/Trc-*maeB*/Trc-*ilvBN*/*gdhA**^{T}), and a monoclonal colony was transferred into an LB test tube containing 0.05 mg/L kanamycin to be cultured at 30°C overnight; the monoclonal colony was inoculated into a 250 mL shake flask containing 50 mL of LB medium at an inoculum amount of 1% (volume concentration), 500 µL of 1 mol/L L-arabinose was added, the product was cultured at 150 rpm and 30°C till OD₆₀₀ was 0.4-0.6; cells were collected after centrifugation at 4,000 rpm and 4°C for 10 minutes, electrocompetent cells were prepared, and details are shown in the description of (Molecular Cloning: A Laboratory Manual, 3ed Edition, 99-102).
(4) An appropriate quantity of pTD*-EcilvD* (about 200 ng) plasmids were pipetted with a pipette and mixed with 100 µL of prepared electroshock competent cells, the mixture was transferred into a precooled 2 mm electroshock cup, an electroporation apparatus (MicroPluser^{™}, BIO-RAD) was used for electroshock transformation after an ice bath for about 1-2 minutes, 800 µL of LB medium was immediately added after electroshock, the product was immediately pipetted gently and transferred into a 2 mL Ep tube, an LB solid plate containing 0.05 mg/L kanamycin and 0.05 mg/L spectinomycin was coated with the product after resuscitation at 30°C for 3-4 hours, inverted culture was performed at 30°C for 12-16 hours, *ilvD-*VF/R was adopted as primers for colony PCR verification, and if a fragment about 3500 bp could be successfully cloned, it was proved that it was a DPAP7 (DPAL6 derivative, *yjiV*::Ptrc-*EcilvD*) positive colony.
(5) Plasmid elimination: Positive single colonies were picked with inoculation loop and inoculated into an LD liquid test tube containing 1 mM IPTG and 0.05 mg/L kanamycin to be cultured overnight at 30°C, a bacterial solution was streaked on an LB solid plate containing 0.05 mg/L kanamycin on the next day to be cultured at 30°C for 24 hours, after bacteria grew to a certain size, part of the single colonies were picked and streaked on an LB plate containing 0.05 mg/L spectinomycin, the plasmids pTarget-*yjiV* of single colonies that could not be in the LB plate containing 0.05 mg/L spectinomycin were successfully eliminated, then the single colonies where the plasmids pTarget-*yjiV* were successfully eliminated were picked on an LB test tube to be cultured overnight at 37°C for eliminating plasmids pCas, a bacterial solution was scribed on an LB plate on the next day to be cultured at 37°C for 12 hours, part of single colonies were picked and streaked on the LB plate containing 0.05 mg/L spectinomycin, the plasmids pCas of single colonies that could not be on the LB plate containing 0.05 mg/L kanamycin were successfully eliminated, and finally a plasmid-free strain DPAP7 (DPAL6 derivative, *yjiV*::Ptrc-*EcilvD*) was obtained.
(6) Shake flask fermentation: DPAP7 (DPAL6 derivative, *yji V*::Ptrc-*EcilvD*) was respectively inoculated into a 10 mL LB medium to be cultured at 37°C and 200 rpm as a preculture product, with the original strain ZJUTDPAL5 as a control group; after 8-12 hours, 1 mL of the preculture product was inoculated into a 500 mL shake flask containing 50 mL of MS medium at a 2% inoculation amount, and then the preculture product was cultured in a constant-temperature shaker at 30°C and 180 rpm for 48 hours for strain fermentation; 1 mL of fermentation broth was taken after fermentation, 1 mL of fermentation broth was pipetted with a pipette while measuring an OD₆₀₀ value, centrifugation was performed at a room temperature and 12,000 rpm for 3 minutes, a fermentation supernatant was diluted five times, and then a diluted sample was subjected to impurity removal with a hydrophilic filter membrane, HPLC detection was performed according to Example 1, and OD₆₀₀ and D-pantothenic acid content in the fermentation broth supernatant are shown in FIG. 2.

It could be shown from the figure that additionally increasing the copy number of the *ilvD* gene on the genome could not significantly affect the growth of the bacteria, as well as could not promote the improvement of D-pantothenic acid shake flask titer, which was about 2.9 g/L similar to that of the original strain, it was possibly caused by insufficient carbon flux in the pathway as mentioned above, and the strain continued to be transformed correspondingly on this basis in consideration of the following transformation requirement.

LB medium: 10 g/L of peptone, 5 g/L of yeast extract and 5 g/L NaCl, deionized water is adopted as a solvent, and a pH value is natural.

MS medium: 20 g/L of glucose, 16 g/L of (NH₄)₂SO₄, 2 g/L of KH₂PO₄, 0.5 g/L of MgSO₄, 2 g/L of yeast extract, 10 g/L of CaCO₃ and 1 mL/L of microelement solution, deionized water is adopted as a solvent, and a pH value is natural; 10 g/L of calcium carbonate (independently sterilized); and the microelement solution includes the following components: 10 g/L of CuCl₂, 10 g/L of FeSO₄·7H₂O, 1 g/L of ZnSO₄·7H₂O, 0.20 g/L of CuSO₄ and 0.02 g/L NiCl₂·7H₂O, and deionized water is adopted as a solvent.

### Example 3: DPAP8 construction and shake flask fermentation

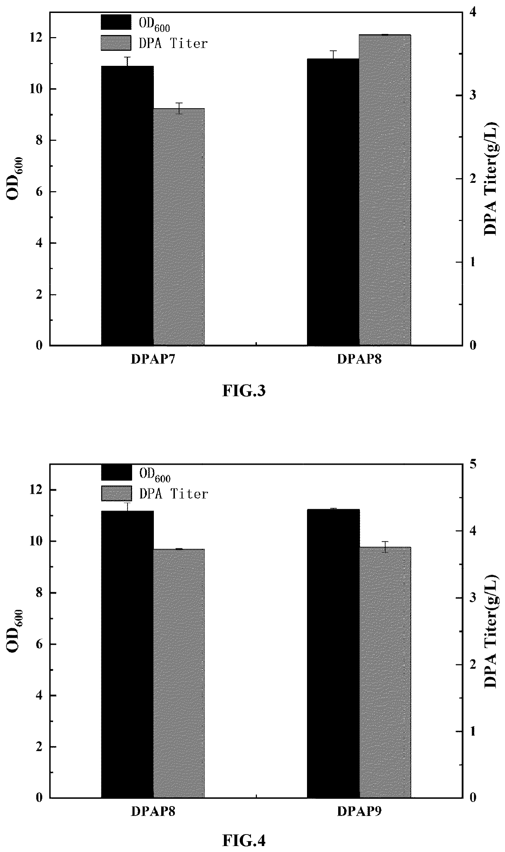

DPAP7 was adopted as an original strain, a CRISPR-Cas9 mediated gene editing technology was applied, and an original pseudogene *flik* on a genome of the original strain was replaced with a *BspanBA* gene (nucleotide sequence was shown in SEQ ID No. 2) regulated by a promoter Ptrc derived from pTrc99A so as to enhance expression intensity of *BspanBA.*
(1) Construction of plasmid pTarget-*flik*: A pTarget F plasmid (Addgene Plasmid #62226) was adopted as a template, pT*-flik-*F/R was adopted as primers for PCR amplification, a nucleic acid PCR product was digested with a *Dpn* I digestive enzyme at a temperature preserved at 37°C for 3 hours after gel electrophoresis verification and then transformed to *E. coli* DH5α, and a correct pTarget-*flik* plasmid was obtained after spectinomycin plate screening and sequencing verification, which was used for ligation with donor DNA subsequently.
(2) Construction of plasmid pTD*-BspanBA:* Firstly, an *E. coli* W3110 genome was adopted as a template, *flik-*S8F/R was adopted as primers for amplification to obtain an upstream portion (F1) of the donor DNA, and flik-X8F/R was adopted as primers for amplification to obtain a downstream portion (F2) of the donor DNA. Then, a *B. subtilis* 168 genome was adopted as a template, pTrc*-BspanBA-*9F/R was adopted as primers for amplification to obtain a *BspanBA* gene fragment (F3) with a promoter pTrc, and PCR fragments were recycled and purified with glue to obtain F1, F2 and F3; the plasmid pTarget-*flik* was subjected to heat preservation at 37°C for 8 hours through *Xba* I and *Pst* I*,* and DNA fragments were recycled with a Clean up kit; the fragments F1, F2 and F3 were ligated into the carrier pTarget-*flik* according to the package insert of ClonExpress^{®} (One step clone kit, Vazyme Biotech, Nanjing, China), and a plasmid pTD*-BspanBA* was obtained after sequencing verification.
(3) A plasmid pCas (Addgene Plasmid #62225) was introduced into DPAP7 competent cells obtained in Example 2, and a preparation method of the DPAP7 electrocompetent cells was the same as that in Example 2 (3).
(4) A DPAP8 positive colony was constructed, and a construction method was the same as that in Example 2 (4).
(5) Plasmid elimination: An implementation method was the same as that in Example 2 (5), and plasmid-free DPAP8 was obtained.
(6) The constructed DPAP8 strain was subjected to shake flask test and detection according to the method in Example 2 (6) with DPAP7 constructed in Example 2 as a control group. OD₆₀₀ and D-pantothenic acid content in a fermentation broth supernatant are shown in FIG. 3.

It could be shown from the figure that after increasing the copy number of the heterogenous *BspanBA* gene on the genome in a gene knockin manner, the growth of bacteria was not affected, however, the D-pantothenic acid shake flask titer was increased to 3.72 g/L, which was possibly caused by the fact that part of carbon flux directed toward branched-chain amino acid was redirected toward the synthesis of pantothenic acid due to gene introduction, thereby promoting the bio-synthesis of the D-pantothenic acid of the strain.

### Example 4: DPAP9 construction and shake flask fermentation

DPAP8 was adopted as an original strain, a CRISPR-Cas9 mediated gene editing technology was applied, and an original pseudogene *ompT* on a genome of the original strain was replaced with a *CgpanC* gene (nucleotide sequence was shown in SEQ ID No. 3) regulated by a promoter Ptrc derived from pTrc99A so as to enhance expression intensity of *CgpanC.*
(1) Construction of plasmid pTarget-ompT: A pTarget F plasmid (Addgene Plasmid #62226) was adopted as a template, pT*-ompT-*F/R was adopted as primers for PCR amplification, a nucleic acid PCR product was digested with a *Dpn* I digestive enzyme at a temperature preserved at 37°C for 3 hours after gel electrophoresis verification and then transformed to *E. coli* DH5α, and a correct pTarget-ompT plasmid was obtained after spectinomycin plate screening and sequencing verification, which was used for ligation with donor DNA subsequently.
(2) Construction of plasmid *pTD-CgpanC:* Firstly, an *E. coli* W3110 genome was adopted as a template, *ompT*-S6F/R was adopted as primers for amplification to obtain an upstream portion (F1) of the donor DNA, and *ompT-*X6F/R was adopted as primers for amplification to obtain a downstream portion (F2) of the donor DNA. Then, a C. *glutamicum* ATCC 13032 genome was adopted as a template, pTrc*-CgpanC-*8F/R was adopted as primers for amplification to obtain a *CgpanC* gene fragment (F3) with a promoter pTrc, and PCR fragments were recycled and purified with glue to obtain F1, F2 and F3; the plasmid pTarget-ompT was subjected to heat preservation at 37°C for 8 hours through *Xba* I and *Pst I,* and DNA fragments were recycled with a Clean up kit; the fragments F1, F2 and F3 were ligated into the carrier pTarget-ompT according to the package insert of ClonExpress^{®} (One step clone kit, Vazyme Biotech, Nanjing, China), and a plasmid pTD*-CgpanC* was obtained after sequencing verification.
(3) A plasmid pCas (Addgene Plasmid #62225) was introduced into DPAP8 competent cells obtained in Example 3, and a preparation method of the DPAP8 electrocompetent cells was the same as that in Example 2 (3).
(4) A DPAP9 positive colony was constructed, and a construction method was the same as that in Example 2 (4).
(5) Plasmid elimination: An implementation method was the same as that in Example 2 (5), and plasmid-free DPAP9 was obtained.
(6) The constructed DPAP8 strain was subjected to shake flask test and detection according to the method in Example 2 (6) with DPAP8 constructed in Example 3 as a control group. OD₆₀₀ and D-pantothenic acid content in a fermentation broth supernatant are shown in FIG. 4.

It could be shown from the figure that after increasing the copy number of the heterogenous BspanBA gene on the genome in a gene knockin manner, the growth of bacteria was not significantly affected, the D-pantothenic acid shake flask titer was slightly increased to 3.76 g/L, however, the increase range was less than that achieved by introducing *BspanB* heterogenously, which was possibly caused by sufficient activity of the gene encoding enzyme on the genome, and the D-pantothenic acid content in a fermentation broth could be significantly increased by further increasing the carbon flux directed toward the synthesis of pantothenic acid.

### Example 5: DPAP10 construction and shake flask fermentation

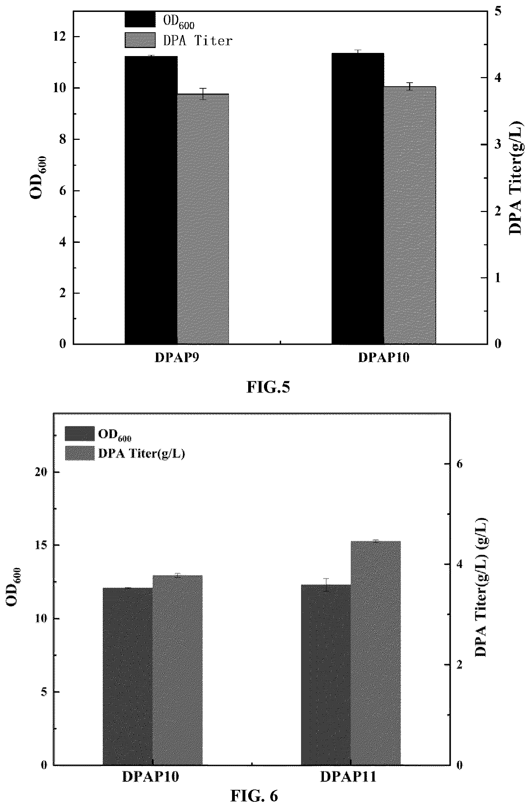

DPAP9 was adopted as an original strain, a CRISPR-Cas9 mediated gene editing technology was applied, and an original pseudogene *yjiP* on a genome of the original strain was replaced with an *alsS* gene (nucleotide sequence was shown in SEQ ID No. 4) regulated by a promoter Ptrc derived from pTrc99A so as to enhance expression intensity of *alsS.*
(1) Construction of plasmid pTarget*-yjiP:* A pTarget F plasmid (Addgene Plasmid #62226) was adopted as a template, pT*-yjiP*-F/R was adopted as primers for PCR amplification, a nucleic acid PCR product was digested with a *Dpn* I digestive enzyme at a temperature preserved at 37°C for 3 hours after gel electrophoresis verification and then transformed to *E. coli* DH5α, and a correct pTarget*-yjiP* plasmid was obtained after spectinomycin plate screening and sequencing verification, which was used for ligation with donor DNA subsequently.
(2) Construction of plasmid pTD-*alsS:* Firstly, an *E. coli* W3110 genome was adopted as a template, *yjiP-*S5F/R was adopted as primers for amplification to obtain an upstream portion (F1) of the donor DNA, and *yjiP*-X5FIR was adopted as primers for amplification to obtain a downstream portion (F2) of the donor DNA. Then, a B. subtilis 168 genome was adopted as a template, pTrc-alsS-17F/R was adopted as primers for amplification to obtain an *alsS* gene fragment (F3) with a promoter pTrc, and PCR fragments were recycled and purified with glue to obtain F1, F2 and F3; the plasmid pTarget*-yjiP* was subjected to heat preservation at 37°C for 8 hours through *Xba* I and *Pst I,* and DNA fragments were recycled with a Clean up kit; the fragments F1, F2 and F3 were ligated into the carrier pTarget*-yjiP* according to the package insert of ClonExpress^{®} (One step clone kit, Vazyme Biotech, Nanjing, China), and a plasmid pTD*-alsS* was obtained after sequencing verification.
(3) A plasmid pCas (Addgene Plasmid #62225) was introduced into DPAP9 competent cells obtained in Example 4, and a preparation method of the DPAP9 electrocompetent cells was the same as that in Example 2 (3).
(4) A DPAP10 positive colony was constructed, and a construction method was the same as that in Example 2 (4).
(5) Plasmid elimination: An implementation method was the same as that in Example 2 (5), and plasmid-free DPAP10 was obtained.
(6) The constructed DPAP10 strain was subjected to shake flask test and detection according to the method in Example 2 (6) with DPAP9 constructed in Example 4 as a control group. OD₆₀₀ and D-pantothenic acid content in a fermentation broth supernatant are shown in FIG. 5.

It could be shown from the figure that after increasing the copy number of the heterogenous *alsS* gene on the genome in a gene knockin manner, the D-pantothenic acid shake flask titer was increased to 3.87 g/L, the effect was insignificant, and therefore the supply of pyruvate precursors or the synthesis capacity of a downstream D-pantothenic acid pathway was still insufficient and needed to be further enhanced.

### Example 6: DPAP11 construction and shake flask fermentation

DPAP10 was adopted as an original strain, a CRISPR-Cas9 mediated gene editing technology was applied, and an original pseudogene *ydeU* on a genome of the original strain was replaced with a *BspanBB* gene (nucleotide sequence was shown in SEQ ID No. 5) regulated by a promoter Ptrc derived from pTrc99A so as to enhance expression intensity of *BspanBB.*
(1) Construction of plasmid pTarget*-ydeU:* A pTarget F plasmid (Addgene Plasmid #62226) was adopted as a template, pT*-ydeU*-F/R was adopted as primers for PCR amplification, a nucleic acid PCR product was digested with a *Dpn* I digestive enzyme at a temperature preserved at 37°C for 3 hours after gel electrophoresis verification and then transformed to *E. coli* DH5α, and a correct pTarget-*ydeU* plasmid was obtained after spectinomycin plate screening and sequencing verification, which was used for ligation with donor DNA subsequently.
(2) Construction of plasmid pTD*-BspanBB:* Firstly, an *E. coli* W3110 genome was adopted as a template, *ydeU-*S6F/R was adopted as primers for amplification to obtain an upstream portion (F1) of the donor DNA, and *ydeU*-X6F/R was adopted as primers for amplification to obtain a downstream portion (F2) of the donor DNA.
   Then, a *B. subtilis 168* genome was adopted as a template, pTrc*-BspanBB-*9F/R was adopted as primers for amplification to obtain a *BspanBB* gene fragment (F3) with a promoter pTrc, and PCR fragments were recycled and purified with glue to obtain F1, F2 and F3; the plasmid pTarget-*ydeU* was subjected to heat preservation at 37°C for 8 hours through *Xba* I and *Pst* I*,* and DNA fragments were recycled with a Clean up kit; the fragments F1, F2 and F3 were ligated into the carrier pTarget-*ydeU* according to the package insert of ClonExpress^{®} (One step clone kit, Vazyme Biotech, Nanjing, China), and a plasmid pTD*-BspanBB* was obtained after sequencing verification.
(3) A plasmid pCas (Addgene Plasmid #62225) was introduced into DPAP10 competent cells obtained in Example 5, and a preparation method of the DPAP10 electrocompetent cells was the same as that in Example 2 (3).
(4) A DPAP11 positive colony was constructed, and a construction method was the same as that in Example 2 (4).
(5) Plasmid elimination: An implementation method was the same as that in Example 2 (5), and plasmid-free DPAP11 was obtained.
(6) The constructed DPAP11 strain was subjected to shake flask test and detection according to the method in Example 2 (6) with DPAP10 constructed in Example 5 as a control group. OD₆₀₀ and D-pantothenic acid content in a fermentation broth supernatant are shown in FIG. 6.

It could be shown from the figure that after increasing the copy number of the heterogenous *BspanBB* gene on the genome again in a gene knockin manner, the D-pantothenic acid yield was still significantly increased, which was about 4.45 g/L. Thus, it was shown that part of carbon flux needed to be further directed toward downstream D-pantothenic acid synthesis after integrating the above several genes into the pathway, thereby promoting the increase of the D-pantothenic acid yield.

### Example 7: DPAP12 construction and shake flask fermentation

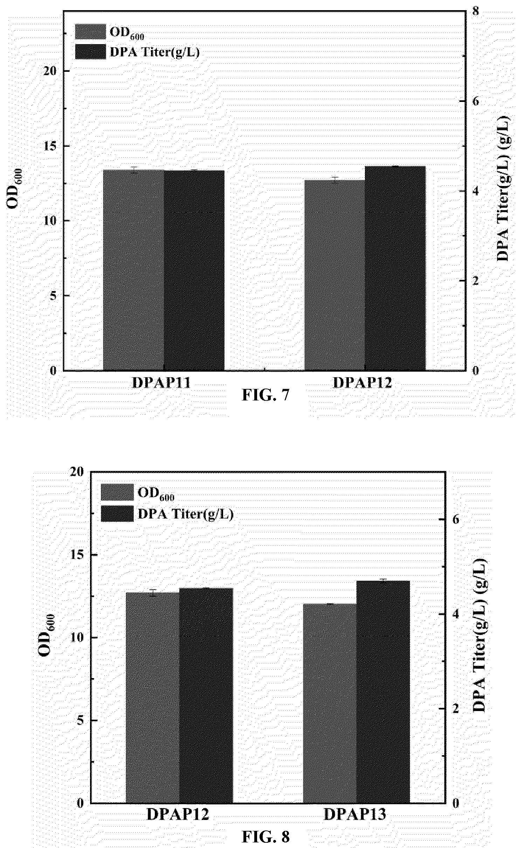

DPAP11 was adopted as an original strain, a CRISPR-Cas9 gene editing technology was applied, a *nac* initiator codon ATG was replaced with GTG (nucleotide sequence was shown in SEQ ID NO. 6) on a genome of the strain DPAP11, and DPAP12 (DPAP11 derivative, *nac*^{GTG}) was obtained.
(1) Construction of plasmid pTarget-*nac*^{GTG}: A plasmid pTarget F (Addgene Plasmid #62226) was adopted as a template, pT*-nac*-F/pT-*nac*-R was adopted as primers for PCR amplification, and a PCR product was digested with *Dpn* I at a temperature preserved at 37°C for 3 hours; and the PCR product of pTarget-*nac*^{GTG} was amplified by linear primers pTarget-XF and pTarget-XR, and a linear carrier pTarget-*nac*^{GTG} was recycled and purified with glue and used for ligation with Donor DNA subsequently.
(2) Construction of plasmid pTD-*nac*^{GTG}: An *E. coli* W3110 genome was adopted as a template, nac-up-F and nac-up-R were adopted as primers for amplification to obtain an upstream portion (F1) of the donor DNA, nac-down-F and nac-down-F were adopted as primers for amplification to obtain a downstream portion (F2) of the donor DNA, and PCR fragments were recycled and purified with glue to obtain F1 and F2; and the fragments F1 and F2 were ligated into the linear carrier pTarget-*nac*^{GTG} according to the package insert of ClonExpress^{®} (One step clone kit, Vazyme Biotech, Nanjing, China), and a plasmid pTD-*nac*^{GTG} was obtained after sequencing verification.
(3) A plasmid pCas (Addgene Plasmid #62225) was introduced into DPAP11 competent cells obtained in Example 6, and a preparation method of the DPAP11 electrocompetent cells was the same as that in Example 2 (3).
(4) A DPAP12 positive colony was constructed, and a construction method was the same as that in Example 2 (4).
(5) Plasmid elimination: An implementation method was the same as that in Example 2 (5), and plasmid-free DPAP12 was obtained.
(6) The constructed DPAP12 strain was subjected to shake flask test and detection according to the method in Example 2 (6) with DPAP11 constructed in Example 6 as a control group. OD₆₀₀ and D-pantothenic acid content in a fermentation broth supernatant are shown in FIG. 7.

It could be shown from the figure that the *nac* initiator codon ATG was replaced with GTG on the genome through gene editing, and the D-pantothenic acid yield was not significantly increased, which was about 4.54 g/L. An *E. coli* nitrogen regulatory protein *Nac* can inhibit the expression of a plurality of genes, including and *cycA, ilvN, ilvH, ilvM* and other genes related to D-pantothenic acid production, under nitrogen limitation. The yield increase range was not wide, which was possibly caused by insufficient *nac* down-regulating degree.

### Example 8: DPAP13 construction and shake flask fermentation

DPAP12 was adopted as an original strain, a CRISPR-Cas9 gene editing technology was applied, in-situ promoters P3, P4 and P5 (nucleotide sequences were shown in SEQ ID NO. 7) located upstream of gene clusters *ptsH, ptsI* and *crr* were knocked out of a genome of the strain DPAP12, and DPAP13 (DPAP12 derivative, ΔP_{345ptsH}) was obtained.
(1) Construction of plasmid pTarget-ΔP_{345ptsH}: A plasmid pTarget F (Addgene Plasmid #62226) was adopted as a template, pT-PptsH-F/pT-PptsH-F was adopted as primers for PCR amplification, and a PCR product was digested with *Dpn* I at a temperature preserved at 37°C for 3 hours; and the PCR product of pTarget-ΔP_{345ptsH} was amplified by linear primers pTarget-XF and pTarget-XR, and a linear carrier pTarget-ΔP_{345ptsH} was recycled and purified with glue and used for ligation with Donor DNA subsequently.
(2) Construction of pTD-ΔP_{345ptsH}: An *E. coli* W3110 genome was adopted as a template, PptsH-up-F and PptsH-up-R were adopted as primers for amplification to obtain an upstream portion (F1) of the donor DNA, PptsH-down-R and PptsH-down-R were adopted as primers for amplification to obtain a downstream portion (F2) of the donor DNA, and PCR fragments were recycled and purified with glue to obtain F1 and F2; and the fragments F1 and F2 were ligated into the linear carrier pTarget-ΔP_{345ptsH} according to the package insert of ClonExpress^{®} (One step clone kit, Vazyme Biotech, Nanjing, China), and a plasmid pTD-ΔP_{345ptsH} was obtained after sequencing verification.
(3) A plasmid pCas (Addgene Plasmid #62225) was introduced into DPAP12 competent cells obtained in Example 7, and a preparation method of the DPAP12 electrocompetent cells was the same as that in Example 2 (3).
(4) A DPAP13 positive colony was constructed, and a construction method was the same as that in Example 2 (4).
(5) Plasmid elimination: An implementation method was the same as that in Example 2 (5), and plasmid-free DPAP13 was obtained.
(6) The constructed DPAP13 strain was subjected to shake flask test and detection according to the method in Example 2 (6) with DPAP12 constructed in Example 7 as a control group. OD₆₀₀ and D-pantothenic acid content in a fermentation broth supernatant are shown in FIG. 8.

It could be shown from the figure that the D-pantothenic yield could be increased to 4.70 g/L by knocking out the in-situ promoters P3, P4 and P5 located upstream the gene clusters *ptsH, ptsI* and *Crr* on the genome. A phosphotransferase system consumes PEP to transfer glucose, PEP is saved by slightly weakening the phosphotransferase system, and PEP produces pyruvic acid and ATP under the action of a *pykAF* gene as much as possible, thereby promoting the increase of the D-pantothenic acid yield in a shake flask.

### Example 9: DPAP14 construction and shake flask fermentation

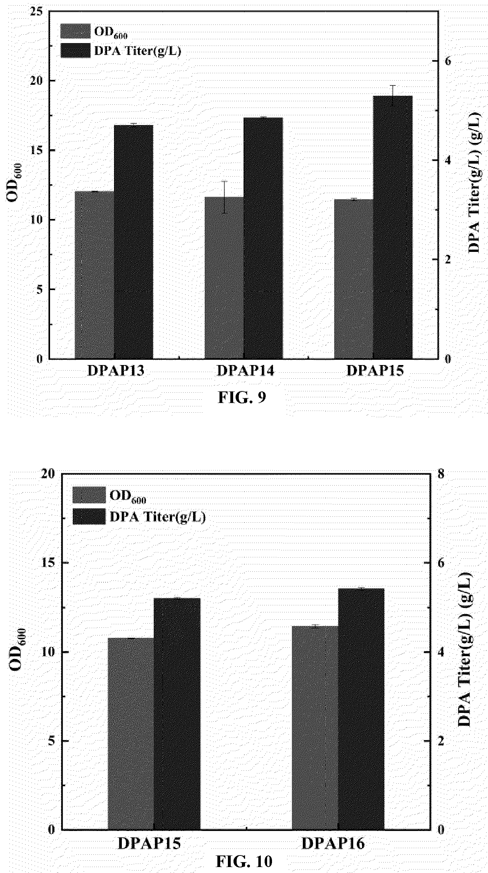

DPAP13 was adopted as an original strain, a CRISPR-Cas9 gene editing technology was applied, a *gltA* initiator codon ATG was replaced with GTG (nucleotide sequence was shown in SEQ ID NO. 8) on a genome of the strain DPAP13, and DPAP14 (DPAP13 derivative, *gltA*^{GTG}) was obtained.
(1) Construction of plasmid pTarget-*gltA*^{GTG}: A plasmid pTarget F (Addgene Plasmid #62226) was adopted as a template, pT-gltA-F/pT-gltA-R was adopted as primers for PCR amplification, and a PCR product was digested with *Dpn* I at a temperature preserved at 37°C for 3 hours; and the PCR product of pTarget-*gltA*^{GTG} was amplified by linear primers pTarget-XF and pTarget-XR, and a linear carrier pTarget-*gltA*^{GTG} was recycled and purified with glue and used for ligation with Donor DNA subsequently.
(2) Construction of plasmid pTD-*gltA*^{GTG}: An *E. coli* W3110 genome was adopted as a template, gltA-up-F and gltA-up-R were adopted as primers for amplification to obtain an upstream portion (F1) of the donor DNA, gltA-gRNA-F and gltA-gRNA-R were adopted as primers for amplification to obtain a midstream portion (F2) of the donor DNA, and gltA-down-F1 and gltA-down-R were adopted as primers for amplification to obtain a downstream portion (F3) of the donor DNA; and the fragments F1, F2 and F3 were ligated into the linear carrier pTarget-*gltA*^{GTG} according to the package insert of ClonExpress^{®} (One step clone kit, Vazyme Biotech, Nanjing, China), construction steps were the same as those in Examples 2 (2), and a plasmid pTD-*gltA*^{GTG} was obtained.
(3) A plasmid pCas (Addgene Plasmid #62225) was introduced into DPAP13 competent cells obtained in Example 8, and a preparation method of the DPAP13 electrocompetent cells was the same as that in Example 2 (3).
(4) A DPAP14 positive colony was constructed, and a construction method was the same as that in Example 2 (4).
(5) Plasmid elimination: An implementation method was the same as that in Example 2 (5), and plasmid-free DPAP14 was obtained.
(6) The constructed DPAP14 strain was subjected to shake flask test and detection according to the method in Example 2 (6) with DPAP13 constructed in Example 8 as a control group. OD₆₀₀ and D-pantothenic acid content in a fermentation broth supernatant are shown in FIG. 9.

It could be shown from the figure that after the *gltA* initiator codon ATG was replaced with GTG on the genome through gene editing, the D-pantothenic acid yield was increased to 4.85 g/L. A large quantity of NADH, ATP and some metabolisms are required at a strain growth stage, resulting in a large amount of carbon flux flowing into a TCA cycle. Thus, in consideration of weakening the TCA cycle to enhance the accumulation of pyruvic acid, the strain would grow slowly due to the inactivation of the gene *gltA,* and the initiator codon ATG of the gene *gltA* was replaced with GTG, so as to weaken the TCA cycle to promote the increase of the D-pantothenic acid yield.

### Example 10: DPAP15 construction and shake flask fermentation

DPAP14 was adopted as an original strain, a CRISPR-Cas9 gene editing technology was applied, a *gltA* initiator codon GTG was replaced with TTG (nucleotide sequence was shown in SEQ ID NO. 9) on a genome of the strain DPAP14, and DPAP15 (DPAP14 derivative, *gltA*^{TTG}) was obtained.
(1) Construction of plasmid pTarget-*gltA*^{TTG}: A plasmid pTarget F (Addgene Plasmid #62226) was adopted as a template, pT-gltA-F/pT-gltA-R was adopted as primers for PCR amplification, and a PCR product was digested with *Dpn* I at a temperature preserved at 37°C for 3 hours; and the PCR product of pTarget-*gltA*^{TTG} was amplified by linear primers pTarget-XF and pTarget-XR, and a linear carrier pTarget-*gltA*^{TTG} was recycled and purified with glue and used for ligation with Donor DNA subsequently.
(2) Construction of plasmid pTarget-*gltA*^{TTG}: An *E. coli* W3110 genome was adopted as a template, gltA-up-F and gltA-up-R were adopted as primers for amplification to obtain an upstream portion (F1) of the donor DNA, gltA-gRNA-F and gltA-gRNA-R were adopted as primers for amplification to obtain a midstream portion (F2) of the donor DNA, and gltA-down-F2 and gltA-down-R were adopted as primers for amplification to obtain a downstream portion (F3) of the donor DNA; and the fragments F1, F2 and F3 were ligated into the linear carrier pTarget-*gltA*^{TTG} according to the package insert of ClonExpress^{®} (One step clone kit, Vazyme Biotech, Nanjing, China), construction steps were the same as those in Examples 2 (2), and a plasmid pTD-*gltA*^{TTG} was obtained.
(3) A plasmid pCas (Addgene Plasmid #62225) was introduced into DPAP14 competent cells obtained in Example 9, and a preparation method of the DPAP14 competent cells was the same as that in Example 2 (3).
(4) A DPAP15 positive colony was constructed, and a construction method was the same as that in Example 2 (4).
(5) Plasmid elimination: An implementation method was the same as that in Example 2 (5), and plasmid-free DPAP15 was obtained.
(6) The constructed DPAP15 strain was subjected to shake flask test and detection according to the method in Example 2 (6) with DPAP13 constructed in Example 9 as a control group. OD₆₀₀ and D-pantothenic acid content in a fermentation broth supernatant are shown in FIG. 9.

It could be shown from the figure that after the *gltA* initiator codon GTG was replaced with TTG on the genome through gene editing, the D-pantothenic acid yield was increased to 5.16 g/L. In consideration of weakening the TCA cycle to replace the initiator codon ATG of the gene *gltA* with GTG, cell growth was not significantly affected, and therefore the initiator codon of the gene *gltA* was further downregulated into TTG, thereby promoting the increase of the D-pantothenic acid yield.

### Example 11: DPAP16 construction and shake flask fermentation

DPAP15 was adopted as an original strain, a CRISPR-Cas9 gene editing technology was applied, and an initiator codon of a *pfkB* gene was replaced with Ptrc (nucleotide sequence was shown in SEQ ID NO. 10) on a genome of the strain DPAP15, and DPAP16 (DPAP15 derivative, PpfkB::Ptrc) was obtained.
(1) Construction of plasmid pTarget-PpfkB::Ptrc: A plasmid pTarget F (Addgene Plasmid #62226) was adopted as a template, pT-PpfkB-F/pT-PpfkB-R was adopted as primers for PCR amplification, and a PCR product was digested with *Dpn* I at a temperature preserved at 37°C for 3 hours; and the PCR product of pTarget-PpfkB::Ptrc was amplified by linear primers pTarget-XF and pTarget-XR, and a linear carrier pTarget-PpfkB::Ptrc was recycled and purified with glue and used for ligation with Donor DNA subsequently.
(2) Construction of plasmid pTD-PpfkB::Ptrc: An *E. coli* W3110 genome was adopted as a template, PpfkB-up-F and PpfkB-up-R were adopted as primers for amplification to obtain an upstream portion (F1) of the donor DNA, PpfkB-down-FF and PpfkB-down-R were adopted as primers for amplification to obtain a downstream portion (F2) of the donor DNA, the fragments F1 and F2 were ligated into the linear carrier pTarget-PpfkB::Ptrc according to the package insert of ClonExpress^{®} (One step clone kit, Vazyme Biotech, Nanjing, China), construction steps were the same as those in Example 2 (2), and a plasmid pTD-PpfkB::Ptrc was obtained.
(3) A plasmid pCas (Addgene Plasmid #62225) was introduced into DPAP15 competent cells obtained in Example 10, and a preparation method of the DPAP15 competent cells was the same as that in Example 2 (3).
(4) A DPAP16 positive colony was constructed, and a construction method was the same as that in Example 2 (4).
(5) Plasmid elimination: An implementation method was the same as that in Example 2 (5), and plasmid-free DPAP16 was obtained.
(6) The constructed DPAP16 strain was subjected to shake flask test and detection according to the method in Example 2 (6) with DPAP15 constructed in Example 10 as a control group. OD₆₀₀ and D-pantothenic acid content in a fermentation broth supernatant are shown in FIG. 10.

It could be shown from the figure that after the initiator codon of the gene *pfkB* was replaced with Ptrc on the genome through gene editing, the D-pantothenic acid yield was increased to 5.43 g/L. Due to the insufficient expression amount of the original gene *pfkB*, the expression amount was increased through a strong initiator, and a glycolysis front-end pathway was enhanced, thereby promoting the increase of the D-pantothenic acid yield.

### Example 12: Fermentation of strains DPAP11 and DPAP16 for producing D-pantothenic acid in 5 L bio-reactor

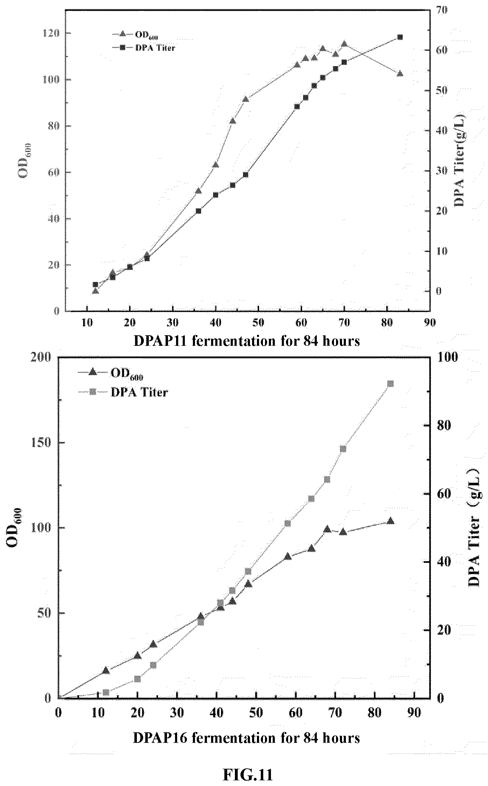

Fermentation was performed in a 5 L fermentation tank (Shanghai Baoxing, BIOTECH-5BG), including the following steps:
(1) Seed culture: A plate was inoculated into 10 mL of LB medium and cultured on a shaker at 37°C and 180 rpm overnight, and then the product was inoculated into two flasks respectively filled with 100 mL of LB media as secondary seed solutions at 1% (volume concentration) to be cultured for 7-12 hours.
(2) Inoculation fermentation: A volume of the fermentation medium in the 5 L fermentation tank was 2 L, and sterilization was performed at 115°C for 30 minutes. The operation was performed at 25-30°C, 3-6 L/min (initial ventilation amount) and 300-450 rpm (initial stirring speed), and pH was adjusted with aqueous ammonia. The two flasks of secondary seed solutions (200 mL) were transferred into a resistance-free 2 L fermentation medium in the 5 L fermentation tank, meanwhile, IPTG with a final concentration of 0.2 mM, VB₁ with a final concentration of 5 mg/L, VB₁₂ with a final concentration of 2 mg/L, and 5-10 mL of isoleucine with a concentration of 10-40 g/L were added, and fermentation was started. Dissolved oxygen was maintained at 10-30% at a dissolved oxygen series rotating speed during fermentation, pH was maintained at 6.7-6.9 with aqueous ammonia as a neutralizer, a glucose concentration in the tank was controlled below 5 g/L through pH linked feeding, culture was performed at 28-37°C for 3-4 days, and a fermentation broth was obtained. The fermentation broth was all substances in the fermentation tank.
(3) After a diluted sample was subjected to impurity removal with a hydrophilic filter membrane after a fermentation supernatant was diluted 80 times, HPLC detection was performed according to Example 1, and OD₆₀₀ and D-pantothenic acid content in the fermentation broth supernatant were shown in FIG. 11.
   It could be shown from the figure that after a D-pantothenic acid pathway was enhanced, the D-pantothenic acid yield was increased to 63.3 g/L after the strain DPAP11 for producing the D-pantothenic acid was fermented in the 5 L fermentation tank for 84 hours. On this basis, the supply of pyruvate precursors was optimized, a TCA cycle was weakened, more carbon flux was promoted to enter the pantothenic acid pathway to obtain the strain DPAP16 for producing the D-pantothenic acid, the D-pantothenic acid yield was increased to 94.2 g/L after fermentation in the 5 L fermentation tank for 84 hours, the synthesis of the D-pantothenic acid was greatly improved, and the fermentation period was shortened.
(4) The feeding medium includes the following components: 20 g/L of glucose, 16 g/L of ammonium sulfate, 2 g/L of betaine anhydrous, 2 g/L of yeast powder, 2 g/L of potassium dihydrogen phosphate, 0.5 g/L of anhydrous magnesium sulfate, 1.5 g/L of beta-alanine and 1 mL/L of microelement solution, deionized water is adopted as a solvent, and a pH value is natural. The microelement solution includes the following components: 10 g/L of CuCl₂, 10 g/L of FeSO₄·7H₂O, 10 g/L of ZnSO₄·7H₂O, 0.2 g/L of CuSO₄ and 0.02 g/L of NiCl₂·-7H₂O, and deionized water is adopted as a solvent.
(5) The feeding medium includes the following components: 500 g/L of glucose, 10 g/L of ammonium sulfate, 4 g/L of betaine anhydrous, 2 g/L of yeast powder, 14 g/L of potassium dihydrogen phosphate, 8 g/L of anhydrous magnesium sulfate, 60 g/L of beta-alanine and 2 mL/L of microelement solution, deionized water is adopted as a solvent, and a pH value is natural.

## Claims

1. Genetically engineered bacteria for producing D-pantothenic acid, constructed through the following method comprising:
(1) adopting genetically engineered bacteria ZJUTDPAL5 as chassis bacteria, increasing the copy number of an *EcilvD* gene regulated by a promoter pTrc on a genome of the genetically engineered bacteria, and obtaining an engineered bacteria DPAL6 derivative, *yjiV*::Ptrc*-EcilvD* and denoting the same as engineered bacteria DPAP7;
(2) increasing the copy number of a bacillus subtilis *BspanBA* gene regulated by the promoter pTrc on a genome of the engineered bacteria DPAP7 in a gene knockin manner, and obtaining an engineered bacteria DPAP7 derivative, *flik*::Ptrc-*BspanBA* and denoting the same as engineered bacteria DPAP8;
(3) increasing the copy number of a corynebacterium glutamicum *CgpanC* gene regulated by the promoter pTrc on a genome of the engineered bacteria DPAP8 in a gene knockin manner, and obtaining an engineered bacteria DPAP8 derivative, *ompT*::Ptrc-*CgpanC* and denoting the same as engineered bacteria DPAP9;
(4) increasing the copy number of a bacillus subtilis *alsS* gene regulated by the promoter pTrc on a genome of the engineered bacteria DPAP9 in a gene knockin manner, and obtaining an engineered bacteria DPAP9 derivative, *yjiP*::Ptrc*-alsS* and denoting the same as engineered bacteria DPAP10;
(5) further increasing the copy number of a bacillus subtilis *BspanBB* gene regulated by the promoter pTrc on a genome of the engineered bacteria DPAP10 in a gene knockin manner, and obtaining an engineered bacteria DPAP10 derivative, *ydeU::Ptrc-BspanBB* and denoting the same as engineered bacteria DPAP11;
(6) replacing an initiator codon ATG of a *nac* gene in a genome of the engineered bacteria DPAP11 with GTG, and obtaining an engineered bacteria DPAP11 derivative, *nac*^{GTG} and denoting the same as engineered bacteria DPAP12;
(7) knocking out in-situ promoters P₃, P₄ and P₅ located upstream of gene clusters *ptsH, ptsI* and *crr* in a genome of the engineered bacteria DPAP12, and obtaining an engineered bacteria DPAP12 derivative, ΔP_{345ptsH} and denoting the same as engineered bacteria DPAP13;
(8) replacing an initiator codon ATG of a *gltA* gene in a genome of the engineered bacteria DPAP13 with GTG, and obtaining an engineered bacteria DPAP13 derivative, *gltA*^{GTG} and denoting the same as engineered bacteria DPAP14;
(9) replacing an initiator codon GTG of a *gltA* gene in a genome of the engineered bacteria DPAP14 with TTG, and obtaining an engineered bacteria DPAP14 derivative, *gltA*^{TTG} and denoting the same as engineered bacteria DPAP15; and
(10) replacing an in-situ promoter of a *pfkB* gene in a genome of the engineered bacteria DPAP15 with Ptrc, and obtaining an engineered bacteria DPAP15 derivative, PpfkB::Ptrc and denoting the same as engineered bacteria DPAP16, namely the genetically engineered bacteria for producing the D-pantothenic acid.

2. The genetically engineered bacteria according to claim 1, wherein nucleotide sequences of the *EcilvD, BspanBA, CgpanC, BspanBB* and *alsS* genes regulated by the promoter Ptrc are respectively shown in SEQ ID NO. 1-5, a nucleotide sequence of *nac*^{GTG} is shown in SEQ ID NO. 6, nucleotide sequences of the in-situ promoters P₃, P₄ and P₅ located upstream of the gene clusters *ptsH, ptsI* and *crr* are shown in SEQ ID NO. 7, a nucleotide sequence of *gItA*^{GTG} is shown in SEQ ID NO. 8, a nucleotide sequence of *gltA*^{TTG} is shown in SEQ ID NO. 9, and a nucleotide sequence of the in-situ promoter of the *pfkB* gene is shown in SEQ ID NO. 10.

3. A construction method of the genetically engineered bacteria according to claim 1 or 2, comprising:
(1) adopting genetically engineered bacteria ZJUTDPAL5 as chassis bacteria, applying a CRISPR-Cas9 mediated gene editing technology, replacing an original pseudogene *yjiV* on a genome of an original strain with the *EcilvD* gene regulated by a promoter Ptrc derived from pTrc99A, so as to enhance expression intensity of *EcilvD,* and obtaining an engineered bacteria DPAL6 derivative, *yjiV*::Ptrc-*EcilvD* and denoting the same as engineered bacteria DPAP7;
(2) adopting the engineered bacteria DPAP7 as an original strain, applying the CRISPR-Cas9 mediated gene editing technology, replacing an original pseudogene *flik* on a genome of the original strain with the *BspanBA* gene regulated by the promoter Ptrc derived from pTrc99A, so as to enhance expression intensity of *BspanBA,* and obtaining an engineered bacteria DPAP7 derivative, *flik*::Ptrc*-BspanBA* and denoting the same as engineered bacteria DPAP8;
(3) adopting the engineered bacteria DPAP8 as an original strain, applying the CRISPR-Cas9 mediated gene editing technology, replacing an original pseudogene *ompT* on a genome of the original strain with the *CgpanC* gene regulated by the promoter Ptrc derived from pTrc99A, so as to enhance expression intensity of *CgpanC,* and obtaining an engineered bacteria DPAP8 derivative, *ompT*::Ptrc-*CgpanC* and denoting the same as engineered bacteria DPAP9;
(4) adopting the engineered bacteria DPAP9 as an original strain, applying the CRISPR-Cas9 mediated gene editing technology, replacing an original pseudogene *yjiP* on a genome of the original strain with the *alsS* gene regulated by the promoter Ptrc derived from pTrc99A, so as to enhance expression intensity of *alsS,* and obtaining an engineered bacteria DPAP9 derivative, *yjiP*::Ptrc*-alsS* and denoting the same as engineered bacteria DPAP10;
(5) adopting the engineered bacteria DPAP10 as an original strain, applying the CRISPR-Cas9 mediated gene editing technology, replacing an original pseudogene *ydeU* on a genome of the original strain with the *BspanBB* gene regulated by the promoter Ptrc derived from pTrc99A, so as to enhance expression intensity of *BspanBB,* and obtaining an engineered bacteria DPAP10 derivative, *ydeU*::Ptrc-*BspanBB* and denoting the same as engineered bacteria DPAP11;
(6) adopting the engineered bacteria DPAP11 as an original strain, applying the CRISPR-Cas9 mediated gene editing technology, replacing an initiator codon ATG of the *nac* gene in a genome of the engineered bacteria DPAP11 with GTG, and obtaining an engineered bacteria DPAP11 derivative, *nac*^{GTG} and denoting the same as engineered bacteria DPAP12;
(7) adopting the engineered bacteria DPAP12 as an original strain, applying the CRISPR-Cas9 mediated gene editing technology, knocking out in-situ promoters P3, P4 and P5 located upstream of gene clusters *ptsH, ptsI* and *crr* in a genome of the engineered bacteria DPAP12, and obtaining an engineered bacteria DPAP12 derivative, ΔP_{345ptsH} and denoting the same as engineered bacteria DPAP13;
(8) adopting the engineered bacteria DPAP13 as an original strain, applying the CRISPR-Cas9 mediated gene editing technology, replacing an initiator codon ATG of the *gltA* gene in a genome of the engineered bacteria DPAP13 with GTG, and obtaining an engineered bacteria DPAP13 derivative, *gltA*^{GTG} and denoting the same as engineered bacteria DPAP14;
(9) adopting the engineered bacteria DPAP14 as an original strain, applying the CRISPR-Cas9 mediated gene editing technology, replacing an initiator codon GTG of the *gltA* gene in a genome of the engineered bacteria DPAP14 with TTG, and obtaining an engineered bacteria DPAP14 derivative, *gltA*^{TTG} and denoting the same as engineered bacteria DPAP15; and
(10)adopting the engineered bacteria DPAP15 as an original strain, applying the CRISPR-Cas9 mediated gene editing technology, replacing an in-situ promoter of the *pfkB* gene in a genome of the engineered bacteria DPAP15 with Ptrc, and obtaining an engineered bacteria DPAP15 derivative, PpfkB::Ptrc and denoting the same as engineered bacteria DPAP16, namely the genetically engineered bacteria for producing the D-pantothenic acid.

4. The construction method of the genetically engineered bacteria according to claim 3, wherein nucleotide sequences of the *EcilvD, BspanBA, CgpanC, BspanBB* and *alsS* genes regulated by the promoter Ptrc are respectively shown in SEQ ID NO. 1-5, a nucleotide sequence of the *nac*^{GTG} is shown in SEQ ID NO. 6, nucleotide sequences of the in-situ promoters P₃, P₄ and P₅ located upstream of the gene clusters *ptsH, ptsI* and *crr* are shown in SEQ ID NO. 7, a nucleotide sequence of the *gltA*^{GTG} is shown in SEQ ID NO. 8, a nucleotide sequence of the *gltA*^{TTG} is shown in SEQ ID NO. 9, and a nucleotide sequence of the *pfkB* gene in-situ promoter is shown in SEQ ID NO. 10.

5. Application of the genetically engineered bacteria according to claim 1 or 2 in preparation of D-pantothenic acid through microbial fermentation.

6. The application according to claim 5, wherein the application is as follows: Inoculating the genetically engineered bacteria for producing the D-pantothenic acid into a fermentation medium, performing fermentation culture at 28-37°C and 300-450 rpm for 72-96 hours, taking a supernatant of a fermentation broth after fermentation for separation and purification, and obtaining the D-pantothenic acid.

7. The application according to claim 6, wherein the fermentation medium comprises the following components: 10-30 g/L of glucose, 10-25 g/L of ammonium sulfate, 1-5 g/L of betaine anhydrous, 1-5 g/L of yeast powder, 1-5 g/L of potassium dihydrogen phosphate, 0.5-2 g/L of anhydrous magnesium sulfate, 1-5 g/L of beta-alanine and 1-5 mL/L of microelement solution, deionized water is adopted as a solvent, and a pH value is natural; and the microelement solution comprises the following components: 10 g/L of CuCl₂, 10 g/L of FeSO₄·7H₂O, 10 g/L of ZnSO₄·7H₂O, 0.2 g/L of CuSO₄ and 0.02 g/L of NiCl₂·7H₂O, and deionized water is adopted as a solvent.

8. The application according to claim 6, wherein a method comprises: Filling a 5 L fermentation tank with the fermentation medium with a volume of 1-3 L, sterilizing at 115°C for 30 minutes, inoculating a strain of the genetically engineered bacteria into the fermentation medium with the volume of 1-3 L, performing fermentation culture at 28-37°C, initial ventilation capacity of 3-6 L/min, and initial stirring speed of 300-450 rpm, regulating pH with aqueous ammonia, and meanwhile, adding IPTG with a final concentration of 0-0.4 mM, VB₁ with a final concentration of 5 mg/L, VB₁₂ at 2 mg/L and 5-10 mL of isoleucine at 10-40 g/L; and maintaining dissolved oxygen at 10-30% at a dissolved oxygen series rotating speed during fermentation, maintaining pH at 6.7-6.9 with aqueous ammonia as a neutralizer, adding a feeding medium into the tank through pH linked feeding, controlling a glucose concentration below 5 g/L, culturing at 28-37°C for 72-96 hours, obtaining the fermentation broth, taking the supernatant of the fermentation broth for separation and purification, and obtaining the D-pantothenic acid.

9. The application according to claim 8, wherein the feeding medium comprises the following components: 500 g/L of glucose, 5-25 g/L of ammonium sulfate, 2-8 g/L of betaine anhydrous, 1-5 g/L of yeast powder, 10-20 g/L of potassium dihydrogen phosphate, 5-15 g/L of anhydrous magnesium sulfate, 40-100 g/L of beta-alanine and 1-5 mL/L of microelement solution, deionized water is adopted as a solvent, and a pH value is natural.
